(19) Europäisches Patentamt
European Patent Office
Office européen des brevets

(11) Veröffentlichungsnummer: **0 316 016 A2**

# (12) EUROPÄISCHE PATENTANMELDUNG

(21) Anmeldenummer: **88118869.2**

(22) Anmeldetag: **11.11.88**

(51) Int. Cl.⁴: **C07H 21/04**

(30) Priorität: **12.11.87 DE 3738460**

(43) Veröffentlichungstag der Anmeldung: **17.05.89 Patentblatt 89/20**

(84) Benannte Vertragsstaaten: **BE CH DE FR GB IT LI NL SE**

(71) Anmelder: **Max-Planck-Gesellschaft zur Förderung der Wissenschaften e.V.**
**Bunsenstrasse 10**
**D-3400 Göttingen(DE)**

(72) Erfinder: **Pieles, Uwe**
**Am Weendespring 24 a**
**D-3400 Göttingen(DE)**
Erfinder: **Englisch, Uwe, Dr.**
**Auf der Lieth 36**
**D-3400 Göttingen(DE)**
Erfinder: **Cramer, Friedrich, Prof. Dr.**
**Jacob-Henle-Strasse 1 8**
**D-3400 Göttingen(DE)**

(74) Vertreter: **Huber, Bernhard, Dipl.-Chem. et al**
**Möhlstrasse 22 Postfach 860 820**
**D-8000 München 86(DE)**

## (54) Modifizierte oligonukleotide.

(57) Neue modifizierte Oligonukleotide weisen unter den ersten vier Basen am 5′-Ende die Basenfolge AT oder TA auf und enthalten gebunden an eine der beiden O⁻-Gruppen der 5′-Phosphatgruppe einen Rest der allgemeinen Formel

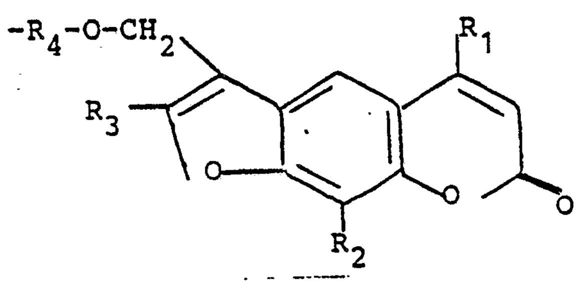

(I)

in der $R_4$ eine Spacergruppe und $R_1$, $R_2$ und $R_3$ H, Alkyl oder Alkoxy mit 1 bis 3 C-Atomen bedeuten. Sie eignen sich zur Blockierung der Replikation oder/und Expression von Genen, die eine mit dem modifizierten Oligonukleotid komplementäre Segenz aufweisen.

EP 0 316 016 A2

# FIG.1

AM 5'-ENDE MODIFIZIERTES DESOXYOLIGONUKLEOTID
MIT KOVALENT GEBUNDENEM PSORALENDERIVAT

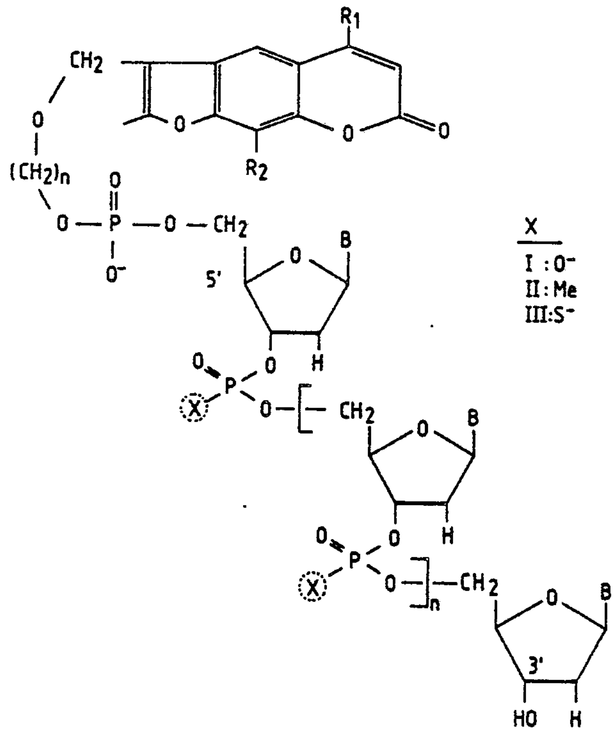

# Modifizierte Oligonukleotide

Die Erfindung betrifft modifizierte Oligonukleotide, ihre Herstellung sowie ihre Verwendung zur Blockierung der Replikation oder/und Expression von Genen.

Eines der großen Ziele der biotechnologischen und medizinischen Forschung ist es heutzutage, die krankhafte Expression bestimmter unerwünschter Genprodukte, die Vermehrung von Viren, sowie die Vermehrung unkontrolliert wuchernder Zellinien, wie dies z. B. bei Krebs oder Psoriasis der Fall ist, zu verhindern.

So wird zur Bekämpfung von bösartigen Hauttumoren vom Typ der sogenannten T-Zell-Lymphome in New England Journal of Medicine, Band 316, Seite 297 vorgeschlagen, bösartige Lymphocyten, die aus dem Blut des Kranken durch Zelltrennverfahren entnommen werden, mit Methoxypsoralen zu behandeln und danach diese Lymphozyten mit UVA-Licht zu bestrahlen. Psoralen und seine Derivate sind zwischen DNA-Doppelstränge interkalierende Verbindungen, die durch Lichteinwirkung an bestimmte Stellen der DNA, nämlich an benachbart gegenüberliegende Thymidinreste kovalent gekoppelt werden. Hierdurch wird im oben dargestellten Fall die DNA der Lymphocyten dauerhaft inaktiviert, worauf diese nach einigen Tagen absterben. Unmittelbar nach der extrakorporalen Bestrahlung erhält der Patient aber das Blut mit den veränderten Lymphozyten zurück, wobei die geschädigten Lymphozyten quasi als Impfung wirken. Das Immunsystem des Körpers wird aktiviert und richtet sich offenbar auch gegen die noch verbliebenen T-Lymphozyten, wodurch sich die tumorösen Hautveränderungen und die Hautrötung zurückbilden. Die Mechanismen, die hierbei ablaufen, sind noch unklar und müssen in Zukunft geklärt werden. Eine ähnliche Behandlung wird auch bereits bei der Psoriasis angewandt.

Diese Form der Behandlung ist jedoch nur möglich, weil man heute einzelne Untergruppen von Blutzellen, in diesem Fall bösartige T-Lymphozyten, durch Trennverfahren wie die Leukophorese isolieren kann, und weil es extrakorporale Vorrichtungen gibt, in denen man ohne Belastung des Gesamtorganismus Pharmaka und Strahlen auf diese Zellen einwirken lassen kann. Der Nachteil dieses Verfahrens ist jedoch die Unspezifität der Interkalation und Kopplung des Psoralens oder seiner Derivate in die gesamte DNA aller behandelten Zellen an TA-Sequenzen und die damit verbundene Notwendigkeit, kranke zu behandelnde Zellen von gesunden Zellen, die nicht abgetötet werden sollen, abzutrennen. Da eine solche Abtrennung nur selten, wie in dem speziellen, oben geschilderten Beispiel machbar ist, wird es durch dieses Verfahren keineswegs ermöglicht, spezifisch virusinfizierte Zellen zu inaktivieren, und dadurch die Vermehrung des Virus zu stoppen oder die Expression sonstiger unerwünschter zellulärer Genprodukte, wie z.B. der Oncogenprodukte in Krebszellen, zu verhindern.

Eine weitere Methode, die auf demselben Prinzip beruht, wurde in der Europäischen Patentanmeldung Nr. 30,358 vorgeschlagen. Bei dieser Methode wird zur Verminderung der Lymphozytenzahl im Blut von Patienten mit abnormal hohem Lymphozytenspiegel (z. B. Lymphozytenleukämie) den Patienten Blut entnommen, dieses Blut in Anwesenheit von Psoralen mit UV-Licht bestrahlt und nachfolgend das Blut den Patienten wieder zugeführt. Hierdurch werden die Lymphozyten, in deren DNA Psoralen interkaliert ist, zerstört und dadurch die Gesamtzahl der Lymphozyten durch kontrollierte Anwendung der Methode wieder auf ein normales Niveau gebracht. Auch bei dieser Methode ist es jedoch nicht möglich, zwischen virusinfizierten Zellen und nicht infizierten Zellen zu unterscheiden. Auch läuft diese Methode auf eine komplette Zerstörung der Zellen hinaus, jedoch nicht auf die Blockierung der Expression bestimmter Gene. Psoralen und seine Derivate werden weiterhin gemäß der Europäischen Patentanmeldung EP 184,331 verwendet, um Viren, z. B. den HIV-Virus, zur Verwendung in Diagnose-Testpackungen zu inaktivieren. Hierbei wird Psoralen zu virushaltigem Zellüberstand zugegeben, dieser mit UV-Licht bestrahlt und daraus die inaktivierten Viren isoliert. Auch dadurch ergibt sich aber keine spezifische Anwendung einer solchen Methode auf zellulärer Ebene.

Es wurde daher in der US-Appl. 563,939 vorgeschlagen, eine doppelsträngige virale RNA, in diesem Fall von Blue-Tongue-Virus (BTV, einem doppelsträngigen RNA-Virus), die durch lichtinduzierte kovalente Kopplung von Psoralen inaktiviert ist, als Impfstoff gegen diesen Virus zu verwenden. Hierdurch kann jedoch nicht ein bereits in einer Zelle vorhandener, in eine zelluläre DNA integrierter Virus spezifisch aufgefunden und inaktiviert werden. Vielmehr kann eine solche Impfung nur als vorbeugende Maßnahme gegen die Infektion mit bestimmten Viren angewandt werden.

Es wurde auch bereits versucht, Psoralen an eine spezifische Stelle in Plasmid-DNA zu dirigieren, wozu in das Plasmid pBR322 durch Methoden der Nick-Translation Quecksilber-modifizierte Pyrimidine eingebaut wurden (Saffran et al., Proc. Natl. Acad. Sci. USA, Vol. 79, S. 4594-4558). Sodann wurde das Psoralen im Dunklen durch eine Hg-S-Bindung an diese Stelle spezifisch gebunden und danach zur Quervernetzung der

beiden Stränge bestrahlt. Diese Methode der spezifischen Kopplung von Psoralen an DNA kann jedoch nur mit Plasmiden oder dergleichen in vitro durchgeführt werden, da es nicht möglich ist, in zellulärer intakter DNA eine solche Modifikation anzubringen. Außerdem wird bei dieser Methode die Nick-Translation mit einem Restriktionsenzym (BamHI) zusammen mit Ethidiumbromid durchgeführt, was voraussetzt, daß an der Stelle, an die das Psoralen gekoppelt werden soll, eine Schnittstelle für ein Restriktionsenzym vorhanden ist. Diese Nick-Translation ist zudem nicht quantitativ durchführbar, es wird nur etwa an die Hälfte der eingesetzten DNA-Moleküle ein Psoralenmolekül gekoppelt.

Der Erfindung liegt daher die Aufgabe zugrunde, die genannten Nachteile zu vermeiden und Verbindungen bereitzustellen, die es ermöglichen, ein quervernetzendes, interkalierendes Agens an einer spezifischen Stelle in ein Gen auch in der intakten Zelle einzubringen, und somit die Expression dieses Genes zu verhindern oder aber ein quervernetzendes Agens an bereits transkribierte RNA zu binden und dadurch die Expression des Gens auf der Translationsebene zu blockieren.

Diese Aufgabe wird erfindungsgemäß durch modifizierte Oligonukleotide gelöst, die dadurch gekennzeichnet sind, daß sie unter den ersten vier Basen am 5′-Ende die Basenfolge AT oder TA auweisen und gebunden an eine der beiden $0^-$-Gruppen der 5′-Phosphatgruppe ein Rest der allgemeinen Formel

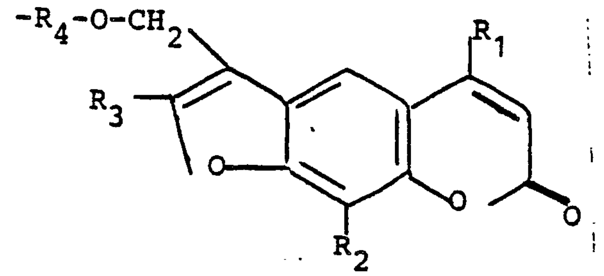

(I)

enthalten, wobei $R_4$ eine Spacergruppe und $R_1$, $R_2$ und $R_3$ H, Alkyl oder Alkoxy mit 1 bis 3 C-Atomen bedeuten.

Die erfindungsgemäßen modifizierten Oligonukleotide hybridisieren mit zu ihnen komplementärer, einzelsträngiger oder doppelsträngiger DNA oder RNA, wobei die interkalierende Verbindung durch das Vorhandensein der Basenfolge AT oder TA auf dem Oligonukleotid in die Nähe einer $^{AT}_{TA}$ -Basenfolge im Doppelstrang dirigiert wird. Bei doppelsträngiger DNA erfolgt eine Hybridisierung des Oligonukleotids nach partiellem Aufschmelzen des ursprünglichen Doppelstrangs (C. Helene et al., Biochemie 67, 777-783 (1985)) oder durch Bildung einer "Triplehelix" (T. Le Doan et al., Nucl. Acids. Res. 15, 7749-7760 (1987)). Nach der Interkalation des Restes der allgemeinen Formel I zwischen die beiden Hybrid-Nukleinsäurestränge (DNA/DNA oder DNA/RNA) werden die Stränge nach Bestrahlung mit UV-Licht über kovalente Bindung der beiden Thymidinbasen an den Rest der Formel I quervernetzt.

In einer bevorzugten Ausführungsform der Erfindung trägt das Oligonukleotid anstelle der zweiten $0^-$-Gruppe der 5′-Phosphatgruppe oder/und an einer oder mehreren weiteren Phosphatgruppen des Oligonukleotids einen Methyl- oder Thiorest. Hierdurch wird es ermöglicht, daß ein Oligonukleotid durch die Zellmembran in eine eukaryontische Zelle eindringen kann (Miller et al., Biochimie 67 (1985), 769-776; Marcus-Sekura et al., Nucl. Acids Res. Vol. 15 Nr. 14 (1987), S. 5749 ff). Für die Löslichkeit des Oligonukleotids ist es jedoch notwendig, daß an mindestens einer Phosphatgruppe im Oligonukleotid die $0^-$-Gruppe erhalten bleibt. Eine allgemeine Formel der erfindungsgemäßen Oligonukleotide ist in Fig. 1 dargestellt.

In einer weiteren bevorzugten Ausführungsform der Erfindung ist die Spacergruppe $R_4$ ein geradkettiger oder verzweigter, substituierter oder unsubstituierter Alkylen- oder Alkenylenrest, wobei die Länge der Spacerkette zwischen der $0^-$-Gruppe des 5′-Phosphats und dem Sauerstoffatom in der 4′-Seitenkette der Verbindung der allgemeinen Formel II 2 bis 4 C-Atome beträgt. Besonders bevorzugt weist dabei die Spacergruppe $R^4$ die Formel

$-(CH_2)_n-$

auf, wobei n eine ganze Zahl von 2 bis 6 ist. Je näher sich die interkalierende Verbindung nach der Hybridisierung der Nukleinsäurestränge nämlich an den gegenüberliegend benachbarten Thymidin-Basen befindet, desto größer ist die Wahrscheinlichkeit, daß die Interkalation an genau dieser Stelle stattfindet, was für eine kovalente Quervernetzung essentiell ist. Eine gewisse Mindestkettenlänge des Spacers ist jedoch für die Beweglichkeit der interkalierenden Verbindung unerläßlich. Am meisten bevorzugt bedeutet

die Spacergruppe $R^4$ im erfindungsgemäßen Oligonukleotid einen Ethylenrest. Außerdem sind bevorzugt die ersten beiden Basen des Oligonukleotids T und A.

In einer weiteren bevorzugten Ausführungsform der Erfindung werden modifizierte Oligonukleotide verwendet, die als Reste $R^1$, $R^2$ und $R^3$ jeweils Methyl und als $R^4$ einen Ethylenrest enthalten.

Die Länge der modifizierten Oligonukleotide wird in bekannter Weise (Szostak et al., (1979) Methods in Enzymol. 68, 419-428) so gewählt, daß eine spezifische Hybridisierung bei geeigneten Temperaturen möglich ist. Dies bedeutet, daß je kürzer, das Oligonukleotid ist, umso tiefer auch die Temperatur bei der Hybridisierung mit zellulärer oder extrazellulärer doppelsträngiger oder einzelsträngiger DNA sein muß. Bevorzugt beträgt die Kettenlänge des Oligonukleotids 8 bis 50 Nukleotide.

Die Sequenz der erfindungsgemäßen Oligonukleotide ist abhängig von der Sequenz des damit zu hybridisierenden Gens oder seiner mRNA. Ein solches Gen kann jedes zelluläre, oder extrazelluläre Gen sein, wobei das Oligonukleotid sowohl zu einem Teil der 5′-Region des Gens, der Initiationsregion der Expression, zu einem Teil des Gens selber, oder zu einem Teil des primären Transkripts oder der mRNA komplementär sein kann. Bevorzugt ist das Oligonukleotid zu einem Teil eines Gens komplementär, das ein mutiertes Gen, ein Oncogen oder ein für die Vermehrung eines Virus essentielles Gen ist.

Ein weiterer Gegenstand der Erfindung ist ein Verfahren zur Herstellung der erfindungsgemäßen modifizierten Oligonukleotide, das dadurh gekennzeichnet ist, daß man eine Verbindung der allgemeinen Formel

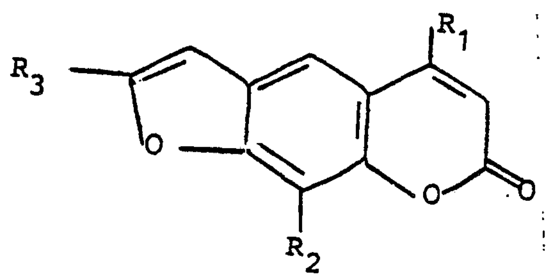

(II)

wobei $R_1$, $R_2$ und $R_2$ H, Alkyl oder Alkoxy mit 1 bis 3 C-Atomen bedeuten, nach an sich bekannten Methoden mit $ClCH_2OCH_3$ und einem zweiwertigen geradkettigen oder verzweigten, substituierten oder unsubstituierten Alkohol unter Bildung einer Verbindung der allgemeinen Formel

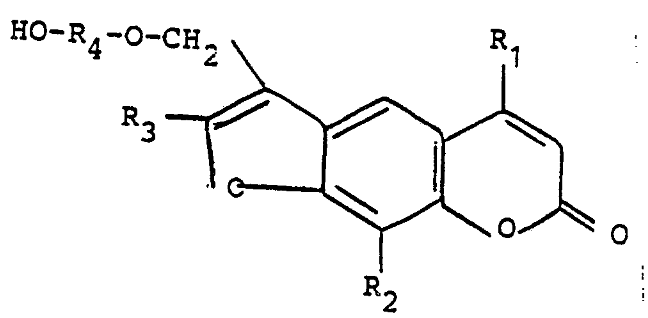

(III)

umsetzt, wobei $R_1$, $R_2$ und $R_3$ die obengenannte Bedeutung besitzen und $R_4$ eine Spacergruppe ist, die Verbindung III mit einer eine geschützte Phosphitgruppe einführenden Verbindung bei Raumtemperatur umsetzt, an das entstandene Reaktionsprodukt mit der allgemeinen Formel

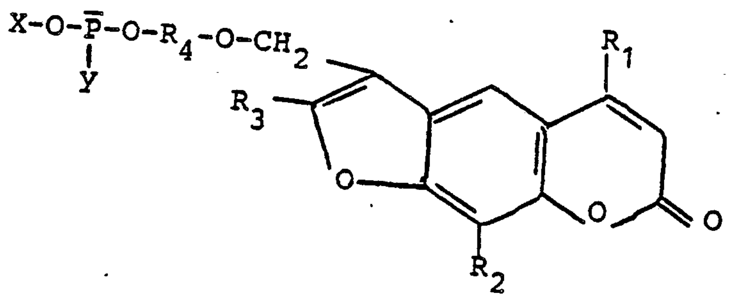

(IV)

wobei $R_1$, $R_2$, $R_3$ und $R_4$ die obengenannte Bedeutung haben und X eine Schutzgruppe und Y eine Cl-, N,N-Dimethylamino-, N,N-Diisopropylamino- oder eine Morpholinogruppe ist, nach an sich bekannten Methoden zur Oligonukleotidsynthese weitere mit einer Schutzgruppe versehene Nukleotide in gewünschter Sequenzfolge ankondensiert, wobei man innerhalb der ersten vier Basen der wachsenden Oligonukleotidkette die Basenfolge AT oder TA einbaut, oxidiert und von den entstandenen Oligonukleotiden die Schutzgruppen durch Hydrolyse entfernt.

Im erfindungsgemäßen Verfahren wird die Verbindung der allgemeinen Formel (II) mit $ClCH_2OCH_3$ umgesetzt, wobei an die 4′- Position im Furanring eine Chlormethylgruppe gebunden wird. Bei Umsetzung mit dem zweiwertigen Alkohol addiert sich dieser an die Chlormethylgruppe unter Abspaltung von HCl, was zu der Verbindung der allgemeinen Formel (III) führt. Diese beiden Umsetzungen sind in der DE-OS 30 33 896 beschrieben. Nach der folgenden Einführung der geschützten Phosphitgruppe wird dann die Verbindung der allgemeinen Formel (IV) als Ausgangsverbindung zur Oligonukleotidsynthese verwendet. Die Phosphitgruppe trägt, gebunden an das Phosphoratom, bevorzugt eine N,N-Diisopropylaminogruppe und gebunden an ein Sauerstoffatom der Phosphitgruppe eine Schutzgruppe, wie sie auch bei der Oligonukleotidsynthese als Schutzgruppe verwendet wird.

Die Oligonukleotidsynthese wird nach an sich bekannten Methoden, z. B. nach Caruthers et al., Science 230 (1980), 281-285, durchgeführt.

Da es, wie bereits erwähnt, für die Spezifität der Einlagerung der interkalierenden Verbindung in den DNA-Doppelstrang wichtig ist, daß sich die Basenfolge $^{TA}_{AT}$ möglichst nah benachbart zur interkalierenden Gruppe befindet, wird innerhalb der ersten vier Basen der wachsenden Nukleotidkette die Basenfolge AT oder TA eingebaut. Die weitere Basenfolge der modifizierten Oligonukleotide wird so gewählt, daß sie zu einem Teil eines Gens, dessen Expression blockiert werden soll, oder dessen mRNA komplementär ist. Nach dem Ankondensieren der gewünschten Oligonukleotidsynthese werden die Phosphitgruppen zu Phosphatgruppen oxidiert und die sich noch an den Phosphatgruppen befindenden Schutzgruppen durch Hydrolyse entfernt.

Bevorzugt wird als eine eine geschützte Phosphitgruppe übertragende Verbindung Monochlor-(β-cyanoethyl-N,N-diisopropylamino)-phosphit verwendet und mit $J_2/H_2O$/Lu tidin oder $S_8/CS_2$/Pyridin oxidiert. Dadurch werden Oligonukleotide hergestellt, die am Phosphoratom der 5′-Phosphatgruppe und eventuell weiteren Phosphatgruppen innerhalb der Oligonukleotidkette je nach Art der Oxidation während der Oligonukleotidsynthese ein negativ geladenes Sauerstoffatom oder/und ein negativ geladenes Schwefelatom und ein doppelt gebundenes Sauerstoffatom enthalten. Bei Durchführung der Oxidation mit $S_8/CS_2$/Pyridin können darüberhinaus auch Oligonukleotide erhalten werden, die an einer oder mehreren Phosphatgruppen anstelle des doppelt gebundenen Sauerstoffatoms ein doppelt gebundenes Schwefelatom enthalten. Die Einführung der geschützten Phosphitgruppe erfolgt wie bei Singha et al., Nucl. Acids. Res. 12 (1984), S. 4539, beschrieben.

In einer weiteren Ausgestaltung der Erfindung wird bevorzugt als eine eine geschützte Phosphitgruppe einführende Verbindung Monochlor-(β-methyl-N,N-diisopropylamino)-phosphit oder/und gegebenenfalls mehrere Nukleosidmethylphosphonamidite zur Oligonukleotidsynthese verwendet und mit $J_2/H_2O$/Lutidin oxidiert. Hierdurch werden gemischte Oligonukleotide, bei denen die Nukleoside teilweise durch Phosphatreste und teilweise durch Methylphosphonatreste verbunden sind, hergestellt. Oligonukleotid-Methylphosphonate oder -Phosphorthioate sind resistent gegen Nukleaseabbau und können die Membranen von Säugerzellen passieren (Miller et al., Biochimie, (1985) 67, Seiten 769-776; Marcus-Secura et al., (1987) Nucl. Acids. Res., Vol. 15, Nr. 14, S. 5749 ff). Das Hybridisationsverhalten zu komplementären Nukleinsäuresequenzen von zellulärer DNA oder RNA ist bei solchen Oligonukleotid-Methylphosphonaten oder -Phosphothioaten nicht beeinträchtigt. Bei reinen Oligonukleotid-Methylphosphonaten ergibt sich jedoch eine Beschränkung ihrer Anwendung durch ihre schlechte Wasserlöslichkeit ab einer Kettenlänge von 8 bis

10 Nukleotiden, weshalb bevorzugt in den erfindungsgemäßen Oligonukleotiden die Nukleoside durch normale Phosphatgruppen und Methylphosphonat oder/und Phosphorthioatgruppen verbunden werden.

Als zweiwertiger Alkohol wird erfindungsgemäß bevorzugt ein geradkettiges oder verzweigtes, substituiertes oder unsubstituiertes Alkandiol oder Alkendiol verwendet.

Besonders bevorzugt wird ein Alkandiol mit der Formel $HO-(CH_2)_n-OH$ verwendet, wobei n eine ganze Zahl von 2 bis 4 ist. Am meisten bevorzugt verwendet man als Alkandiol Ethylenglykol. Hierbei ergibt sich für die Spacergruppe $R^4$ in den Verbindungen der allgemeinen Formel (III) und (IV) die Bedeutung $-CH_2-CH_2-$. In einer weiteren Ausführungsform der Erfindung verwendet man bevorzugt eine Verbindung der allgemeinen Formel (II), bei der die Reste $R^1$, $R^2$ und $R^3$ jeweils einen Methylrest bedeuten und als zweiwertigen Alkohol Ethylenglykol. Die Verbindung der allgemeinen Formel (II) mit dieser Bedeutung der Reste $R^1$ bis $R^3$ ist das 4,5′,8-Trimethylpsoralen.

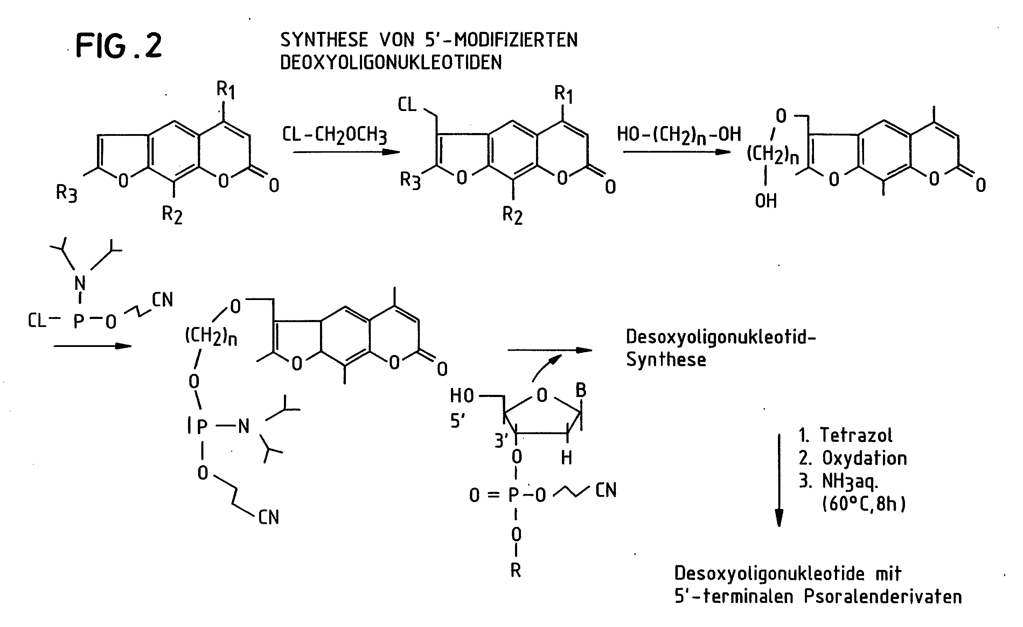

Für eine bevorzugte Ausführungsform der Erfindung ist die Herstellung der modifizierten Oligonukleotide schematisch in Fig. 2 dargestellt.

Um die interkalierende Verbindung der allgemeinen Formel (II) nahe an das Basenpaar T A im Oligonukleotid zu koppeln, wird bevorzugt bei der Oligonukleotidsynthese an die Verbindung der allgemeinen Formel (IV) zuerst ein Thymidin-Monophosphat und dann ein Adenosin-Monophosphat, jeweils in der zur Oligonukleotidsynthese verwendeten geschützten Form, ankondensiert.

Ein weiterer Gegenstand der Erfindung ist die Verwendung von modifizierten Oligonukleotiden, wie sie oben beschrieben sind, zur Blockierung der Expression von Genen, die eine mit dem modifizierten Oligonukleotid komplementäre Sequenz aufweisen.

Hierzu wird das modifizierte Oligonukleotid in Zielzellen, die dieses Gen enthalten, eingebracht und die Zellen mit ultraviolettem Licht bestrahlt.

Durch die erfindungsgemäße Verwendung der modifizierten Oligonukleotide wird es ermöglicht, spezifisch bestimmte Gene, deren Sequenz zumindest teilweise bekannt ist, zu blockieren. Durch Hybridisierung des komplementären Oligonukleotids mit entweder der DNA-Sequenz des zu blockierenden Gens, mit dem primären Transkript oder der mRNA, wird (bei der Doppelstrang-DNA nach partiellem Aufschmelzen des Doppelstrangs) ein Hybriddoppelstrang gebildet und die an das Oligonukleotid gekoppelte interkalierende Verbindung nahe am 5′-Bereich des Hybridstrangs zwischen die Nukleinsäure-Stränge eingelagert. Das modifizierte Oligonukleotid ist hierbei zu einem Bereich des entsprechenden Gens oder der mRNA komplementär, in dem die Basen T und A mindestens einmal benachbart vorkommen. Da das modifizierte Oligonukleotid diese beiden Basen in der komplementären Basenfolge enthält, wird durch die Hybridisierung die interkalierende Verbindung in die Nähe einer solchen Basenfolge gebracht, sie interkaliert in diesem Bereich, und kann durch Bestrahlung bei 360 nm die beiden DNA-Stränge über die benachbart gegenüberliegenden Thymidinreste kovalent verbinden. Durch diese kovalente Verbindung ist es nun nicht mehr möglich, daß sich ein primäres Transkript und daraus eine mRNA zur Transkription des entsprechenden Gens bildet, bzw. das primäre Transkript zu mRNA prozessiert oder mRNA in ein Protein translatiert wird.

Bei der erfindungsgemäßen Blockierung der Expression von Genen kann der Teil des zu blockierenden Gens, zu dem das modifizierte Oligonukleotid komplementär ist, sowohl die 5′-Region des Gens, die Initiationsstelle des Gens, ein Teil des Gens selbst oder das Transkript sein. Durch Bindung an die 5′-Region des Gens und kovalente Quervernetzung der beiden Hybrid-DNA-Stränge in diesen Bereich wird die Promotorwirkung, wie auch die Wirkung weiterer, für die Transkription wichtiger Elemente der Upstream-Region eines Gens gestört. Durch Quervernetzung der Hybrid-DNA-Stränge im Bereich der Initiationsstellen wird die Einwirkung von RNA-Polymerase zum Aufbau eines primären Transkripts verhindert. Durch Quervernetzung der Hybrid-DNA innerhalb des Gens, wird der Abbruch des gebildeten primären Transkripts an dieser Stelle bewirkt. Auch dadurch kann keine intakte mRNA und damit kein intaktes Genprodukt gebildet werden.

Weiter ist es erfindungsgemäß möglich, zu verhindern, daß bereits transkribierte Primärtranskripte oder mRNA in die entsprechenden Proteine translatiert werden. Hierzu ist es jedoch nötig, den Zellen, die, da nicht das Gen blockiert ist, kontinuierlich weiter mRNA bilden können, ständig das modifizierte Oligonukleotid zur Kompensation des primären Transkripts oder der mRNA zuzuführen.

Die erfindungsgemäße Verwendung der modifizierten Oligonukleotide zur Blockierung der Expression von Genen kann sowohl in Eukaryonten, als auch in Prokaryonten durchgeführt werden. In Prokaryonten ist hierbei auch die Blockierung der Expression von auf extrachromosomaler DNA oder RNA (Plasmid, Cosmid, doppelsträngige oder einzelsträngige DNA-Phagen, RNA-Phagen) liegenden Sequenzen möglich.

Bevorzugt wird jedoch die erfindungsgemäße Blockierung der Expression von Genen in Eukaryontenzellen durchgeführt, wobei in einer bevorzugten Ausführungsform der Erfindung die Zielzellen virusinfizierte Zellen sind, und man modifizierte Oligonukleotide verwendet, die zu einem Teil eines für die Virusvermehrung essentiellen Gens komplementär sind. Hierdurch wird es auch ermöglicht, Viren unschädlich zu machen, die ihr Genom in die zelluläre DNA einschleusen, wo es dann latent vorhanden ist, um in einem bestimmten Moment die Virusproduktion zu beginnen. Die Voraussetzung hierzu ist natürlich, daß wenigstens eine Teil-Sequenz des entsprechenden Virus bekannt ist, was jedoch für die meisten Viren der Fall ist. Auch die Blockierung von zellulären, oder viralen, in die Zelle eingeschleusten Onkogenen ist auf diese Art und Weise möglich. Es ist hierdurch vorstellbar, einige Krebsarten, für deren Entstehung bestimmte Retroviren verantwortlich gemacht werden, zu behandeln. Die Voraussetzung hierfür ist nur, daß nach Verabreichung des modifizierten Oligonukleotids die Zielzellen mit UV-Licht bestrahlt werden können. Dies wird bei Menschen und Tieren bevorzugt extrakorporal vorgenommen.

In einer bevorzugten Ausführungsform der Erfindung wird zur Blockierung der Genexpression von Säugetierzellen ein modifiziertes Oligonukleotid verwendet, das mindestens an einer, höchstens jedoch an allen außer einer Phosphatgruppe durch einen Methyl- oder/und Thiorest substituiert ist. Solche modifizierten Oligonukleotide können, wie oben bereits erwähnt, die Zellmembran passieren und damit in das Innere der Zelle gelangen.

Die extrakorporale Bestrahlung von Zellen ist naturgemäß besonders leicht bei Zellen durchzuführen, die sich im menschlichen Blut befinden, da es Dank moderner Apparaturen und medizinischer Behandlungsmethoden möglich ist, Blut teilweise zu entnehmen und nach erfolgter Behandlung dem Körper wieder zuzuführen. Es ist daher eine weitere bevorzugte Ausführungsform der Erfindung, daß man zur Behandlung einer virusinfizierten Leukozytenfraktion bei Menschen und Tieren mit dem erfindungsgemäßen Oligonukleotid nach Verabreichung des Oligonukleotids Blut entnimmt, das Blut extrakorporal bestrahlt und dann wieder dem Körper zuführt. Eine weitere bevorzugte Ausführungsform der Erfindung ist dadurch gekennzeichnet, daß man zur Behandlung von HIV-infizierten Lymphozyten Oligonukleotide verwendet, die zu einem Teil des Reverse Transkriptase-Gens des HIV-Virus komplementär sind, und das Blut der Patienten in mehreren Zyklen nach jeweiliger Teilentnahme extrakorporal bestrahlt, und danach wieder dem Körper zuführt. Dadurch wird in den infizierten Lymphozyten das virale Reverse Transkriptase-Gen blockiert, wodurch eine Umwandlung der DNA des in die Zelle integrierten Provirus in virale RNA nicht mehr möglich und folglich der Vermehrungszyklus des Virus blockiert ist. Es kann daher aus einer solchen Zelle nicht mehr zur Virusvermehrung und damit zur Infektion weiterer Lymphozyten, in diesem Fall der T4-Zellen, kommen. Der Virus wäre hierdurch unschädlich gemacht, obwohl sein Genom weiterhin in das zelluläre Genom integriert bliebe. Aufgrund der heute absolut fehlenden Behandlungsmöglichkeiten für Aids ist diese bevorzugte Anwendung der Erfindung ein wichtiger Schritt zur Bekämpfung der Krankheit, da bereits infizierte Patienten geheilt werden können. Somit blieben auch alle opportunistischen Krankheitserscheinungen, die wegen der Zerstörung der T4-Zellen entstehen können, aus.

Aufgrund der potentiellen Toxizität der interkalierenden Verbindungen wird in einer weiteren bevorzugten Ausführungsform das modifizierte Oligonukleotid nicht oral oder durch Injektion dem Patienten selbst verabreicht, sondern das bereits entnommene Blut wird mit dem modifizierten Oligonukleotid behandelt und sodann extrakorporal bestrahlt. Hierdurch werden mögliche schädliche Auswirkungen des modifizierten Oligonukleotids auf den menschlichen oder tierischen Organismus vermieden.

Durch die erfindungsgemäßen modifizierten Oligonukleotide und ihre Verwendung zur Blockierung der Expression von Genen wird es also ermöglicht, die Expression bestimmter unerwünschter Genprodukte im menschlichen und tierischen Körper spezifisch zu blockieren. Hierzu ist weder eine Manipulation der chromosomalen DNA nötig, noch werden andere, nicht betroffene Gene beeinträchtigt, wie dies bei der unspezifischen Behandlung mit interkalierenden Agentien der Fall ist. Die erfindungsgemäßen modifizierten Oligonukleotide und ihre Verwendung stellen daher einen großen Fortschritt bei der medizinischen Behandlung von Viruserkrankungen oder angeborenen oder entwickeltengenetischen Defekten (z. B. Krebs, Psoriasis) dar.

Die folgenden Beispiele und die Figuren erläutern die Erfindung weiter. Es stellen dar:

Fig. 1 ein erfindungsgemäßes modifiziertes Oligonukleotid,

Fig. 2 schematisch ein bevorzugtes Verfahren zur Herstellung von modifizierten Oligonukleotiden.

**Beispiel 1**

Herstellung eines modifizierten Oligonukleotids

a) 1,38 g (5,74 mmol) 4,5′,8-Trimethylpsoralen werden in 120 ml Eisessig unter leichtem Erwärmen gelöst. Nach Abkühlen auf Raumtemperatur gibt man 10 ml (131,6 mmol) Chlormethylmethylether zu und läßt 24 Stunden bei Raumtemperatur unter Lichtausschluß rühren. Nach 24 Stunden gibt man erneut 10 ml Chlormethylmethylether zu und läßt weitere 24 Stunden rühren. Nun stellt man 12 Stunden auf Eis und saugt anschließend den ausgefallenen Niederschlag ab und trocknet im Hochvakuum bei 40 °C.

Ausbeute: 1,05 g (63,0 %)

$H^1$ -NMR ($CDCl_3$) TMS = 2,6-2,7 (9H,m,C4,5′,8-methyl) 4,8 (2H,s,$CH_2Cl$) 6,3 (1H,s,C3-H) 7,6 (1H,s,C5-H)

b) 360 mg (1,3 mmol) 4-/Chlormethyl/4,5′,8/Trimethylpsoralen wurden dann in 8 ml Ethandiol suspendiert. Danach wurde die Mischung auf 100 °C erhitzt, bis das Psoralenderivat vollständig gelöst war. Es wurde weitere 10 Minuten bei der angegebenen Temperatur gerührt. Nach dem Abkühlen wurde die Mischung mit 10 ml Wasser verdünnt und dreimal mit je 5 ml Dichlormethan extrahiert. Die vereinigten organischen Phasen wurden ber Natriumsulfat getrocknet und nach Abfiltrieren bis zur Trockne eingeengt. Die Reinigung des Rohprodukts erfolgte an Kieselgel. Als Elutionsmittel diente die Dichlormethan/Methanol mit steigendem Methanolgehalt in 0,5% Schritten. Eine ausreichende Reinheit kann aber auch durch Umkristallisation aus Acetonitril erzielt werden. Die Ausbeute betrug 310 mg (78%). Die erhaltene Verbindung wurde über $^1$H-NMR-Spektroskopie nachgewiesen.

$^1$H-NMR ($CDCl_3$) TMS: 2,5 ppm (s, 6H, $-CH_3$), 2,57 (s, 3H, $-CH_3$), 3.6 bis 3.84 (m, 4H, Ethylen), 6.26 (s,m 1H, Pyron), 7.6 (s, 1H, Aromat).

c) 360 mg (1,19 mmol) dieser Verbindung wurden in 10 ml Dichlormethan gelöst und unter Feuchtigkeitsausschluß mit 424 mg (3 mmol) Diisopropylethylamin versetzt. Nun tropfte man langsam 310 mg (1,31 mmol) Monochlor-(ß-cyanoethyl-N,N-diisopropylamino)-phosphit zu. Dann ließ man 30 Minuten bei Raumtemperatur rühren. Nach Abschluß der Reaktion (TLC) wurde durch Zugabe von 5 ml halbgesättigtem Natriumhydrogencarbonat gestoppt. Die Dichlormethanphase wurde noch je zweimal mit halbgesättigtem Natriumhydrogencarbonat gewaschen und anschließend über Natriumsulfat getrocknet. Die Reinigung des Produktes nach Einengen der organischen Phase erfolgte an Kieselgel.
Als Elutionsmittel diente
1. Dichlormethan/Diethylamin 10:1
2. n-Hexan/Triethylamin 10:1.
Die Lösungen 1 und 2 wurden im Verhältnis 1:1 gemischt. Die Ausbeute betrug 419 mg (85%). Die Analyse der Verbindung wurde durch $^{31}$P-NMR Spektroskopie durchgeführt.

$^{31}$P-NMR ($CDCl_3$) $H_3PO_4$ = S 149,54 ppm.

d) Die Kopplung dieses Psoralenphosphoamidids an weitere Nukleotide erfolgte nach Standardbedingungen der Oligonukleotidsynthese nach der Festphasenphosphoamididmethode nach Caruthers (1985) Science <u>230</u> 281-285.

Ablaufdiagramm der Oligonukleotidsynthese am Beispiel der zu der Startsequenz des Lac Z-Gens aus M13 mp19 komplementären Sequenz:
3′AGT GTG TCC TTT GTC GAT X    X = Psoralenderivat

Das Psoralenderivat wird unter den gleichen Bedingungen wie ein normales Nukleotid eingesetzt.

Synthesizer: Modell Sam One Fa. Biosearch Phosphoramidit Nukleotid.

Pro Kupplung werden 40 bis 45 mg des jeweiligen Phosphoramiditnukleosids gelöst in 700 µl trockenem Acetonitril und 24 mg Tetrazol ebenfalls gelöst in 700 µl trockenem Acetonitril eingesetzt.

Als Festphase wird 50 mg Controlled Pore Glass Resin der Firma Pierce verwandt, der nach einer Vorschrift von M.J. Gait, Oligonucleotide-Synthesis, a practical approach, IRL Press (1984), S. 45 ff., mit den entsprechenden Startnucleosiden, hier A, derivatisiert wurde.

Typisches Syntheseprotokoll am Beispiel der ersten Kopplung

| | | |
|---|---|---|
| Wash A | Acetonitril | 1:00 min |
| Capping | Acetanhydrid 7,5 ml<br>Lutidin 7,5 ml<br>N-methylimidazol 1,7 ml<br>in 40 ml Tetrahydrofuran | 1:00 min |
| Wash A | Acetonitril | 1:00 min |
| Deblock | Abspaltung der 5'-Schutzgruppe<br>3% Dichloressigsäure in<br>Dichlorethan | 1:25 min |

| | | |
|---|---|---|
| Wash A | Acetonitril | 1:00 min |
| Wash B | Acetonitril | 1:00 min |
| Couple | Nukleotid wird mit Katalysator gemischt | 0:32 min |
| Mix | Kopplung | 1:30 min |
| Wash A | Acetonitril | 0:30 min |
| Wash B | Acetonitril | 0.30 min |
| Oxidation | 1,5 g Jod in 50 ml Tetrahydrofuran<br>5 ml Wasser und 5 ml Lutidin | 0:45 min |

**Beispiel 2**

Hybridisierung des Psoralen-modifizierten Oligonukleotids mit der Startsequenz des Lac Z-Gens von einzelsträngiger Bakteriophagen-DNA M13 mp19.

50 ng der ss-DNA des Bakteriophagen wurden mit 150 ng des modifizierten Oligonucleotides in einem Volumen von 100 µl hybridisiert. Hierzu erhitzte man die Probe 10 Minuten auf 70° C und ließ anschließend 30 bis 40 Minuten bei 37° C in einem Heizblock abkühlen. Sieben derartig behandelte Proben bestrahlte man nun mit Licht der Wellenlänge 360 nm. Man bestrahlte 0, 1, 3, 5, 10, 20, 30 Minuten. 10 µl der jeweiligen Probe wurden zur Transformation kompetenter Zellen (Calciumchlorid-Methode) eingesetzt. Es wurden E. coli-Zellen des Stammes JM 101 eingesetzt.

Zur Transformation mischte man die 10 µl Probe mit 300 µl kompetenter Zellen und ließ 30 Minuten auf Eis stehen. Währenddessen bereitete man eine Lösung aus 3 ml frischer Zellen ($OD_{600}$ = 0.6) und je 10 mg 5-Bromo-4-chloro-3-indolyl-β-D-galactopyranosid in 500 µl DMF und 10 mg Isopropyl-β-D-thiogalactopyranosid in 500 µl Wasser.

Hiervon gab man je 280 µl zu den Transformationsansätzen, gab dann die gesamte Probe zu 3 ml 42° C warmen H-Topagar und plattierte anschließend auf H-Platten aus. Man inkubierte 18 bis 20 Stunden bei 37° C.

Man beobachtete bei allen derart behandelten Proben ein weitestgehendes Ausbleiben der für die normale Galactosidase-Reaktion typischen blauen Färbung der Plaques, sowie eine drastische Reduzierung der Plaquezahl im Vergleich zu einer unbestrahlten und nicht mit dem modifizierten Oligonukleotid hybridisierten Probe.

**Ansprüche**

1. Modifizierte Oligonukleotide, **dadurch gekennzeichnet**, daß sie unter den ersten vier Basen am 5′-Ende die Basenfolge AT oder TA aufweisen und gebunden an eine der beiden $0^-$ -Gruppen der 5′-Phoshphatgruppe einen Rest der allgemeinen Formel

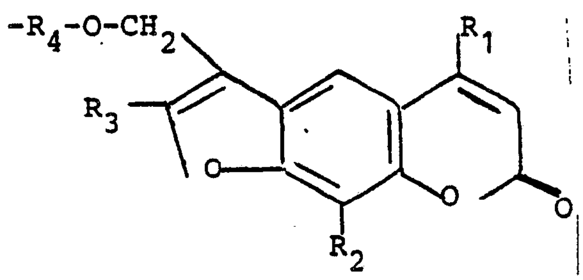

(I)

enthalten, wobei $R_4$ eine Spacergruppe und $R_1$, $R_2$ und $R_3$ H, Alkyl oder Alkoxy mit 1 bis 3 C-Atomen bedeuten.

2. Modifizierte Oligonukleotide nach Anspruch 1, **dadurch gekennzeichnet**, daß die andere $O^-$-Gruppe der 5′-Phosphatgruppe oder/und weitere Phosphatgruppen des Oligonukleotids durch einen Methylrest oder eine $S^-$-Gruppe substituiert sind.

3. Modifizierte Oligonukleotide nach Anspruch 1 oder 2, **dadurch gekennzeichnet**, daß die Spacergruppe $R_4$ ein geradkettiges oder verzweigtes, substituiertes oder unsubstituiertes Alkylen oder Alkenylen mit einer Länge der unverzweigten Kette von 2 bis 6 C-Atomen ist.

4. Modifizierte Oligonukleotide nach Anspruch 3, **dadurch gekennzeichnet**, daß die Spacergruppe $R_4$ die Formel $-(CH_2)_n-$ aufweist, wobei n eine ganze Zahl von 2 bis 6 ist.

5. Modifiziert Oligonukleotide nach Anspruch 4, **dadurch gekennzeichnet**, daß die Spacergruppe $R_4$ die Ethylengruppe ist.

6. Modifizierte Oligonukleotide nach einem der Ansprüche 1 bis 5, **dadurch gekennzeichnet**, daß die Kettenlänge des Oligonukleotids 8 bis 50 Nukleotide beträgt.

7. Modifizierte Oligonukleotide nach einem der Ansprüche 1 bis 6, **dadurch gekennzeichnet**, daß die ersten beiden Basen des Oligonukleotids T und A sind.

8. Modifizierte Oligonukleotide nach einem der Ansprüche 1 bis 7, **dadurch gekennzeichnet**, daß $R_1$, $R_2$ und $R_3$ jeweils Methyl und $R_4$ Ethylen bedeuten.

9. Modifizierte Oligonukleotide nach einem der Ansprüche 1 bis 8, **dadurch gekennzeichnet**, daß die Oligonukleotide zu einem Teil eines Gens komplementär sind, wobei das Gen ein mutiertes Gen, ein Oncogen oder ein für die Vermehrung eines Virus essentielles Gen sein kann.

10. Verfahren zur Herstellung von modifizierten Oligonukleotiden nach einem der Ansprüche 1 bis 9, **dadurch gekennzeichnet**, daß man eine Verbindung der allgemeinen Formel

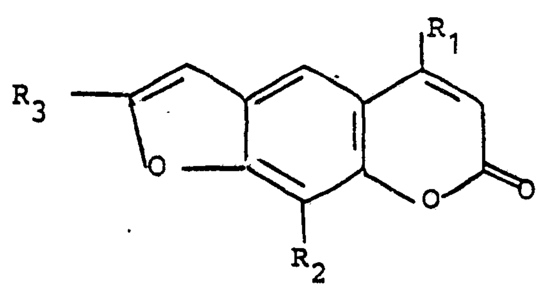

(II)

wobei $R_1$, $R_2$ und $R_3$ H, Alkyl oder Alkoxy mit 1 bis 3 C-Atomen bedeuten, nach an sich bekannten Methoden mit $ClCH_2OCH_3$ und einem zweiwertigen, geradkettigen oder verzweigten, substituierten oder unsubstituierten Alkohol unter Bildung einer Verbindung der allgemeinen Formel

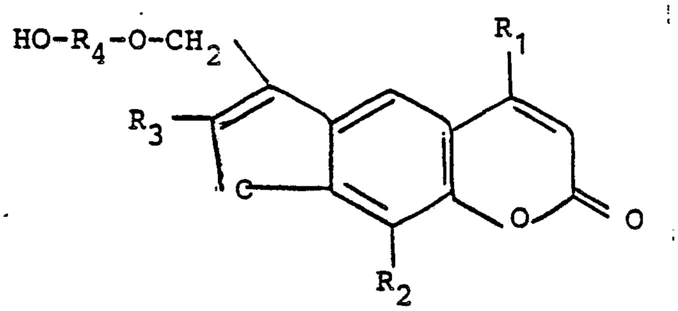

(III)

umsetzt, wobei $R_1$, $R_2$ und $R_3$ die obengenannte Bedeutung besitzen und $R_4$ eine Spacergruppe ist, die Verbindung III mit einer eine geschützte Phosphitgruppe einführenden Verbindung bei Raumtemperatur umsetzt, an das entstandene Reaktionsprodukt mit der allgemeinen Formel

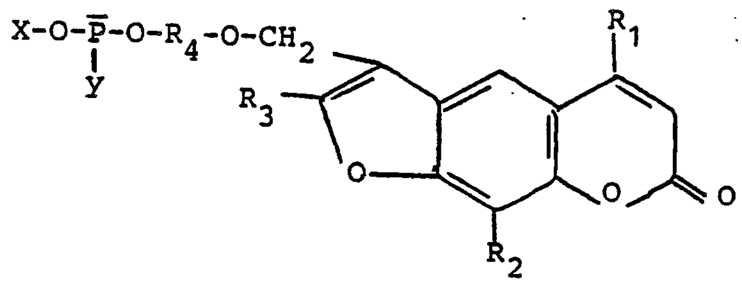

(IV)

wobei $R_1$, $R_2$, $R_3$ und $R_4$ die obengenannte Bedeutung haben und X eine Schutzgruppe und Y eine Cl-, N,N-Dimethylamino-, N,N-Diisopropylamino- oder eine Morpholinogruppe ist, nach an sich bekannten Methoden zur Oligonukleotidsynthese weitere mit einer Schutzgruppe versehene Nukleotide in gewünschter Sequenzfolge ankondensiert, wobei man innerhalb der ersten vier Basen der wachsenden Oligonukleotidkette die Basenfolge AT oder TA einbaut, oxidiert und von den entstandenen Oligonukleotiden die Schutzgruppen durch Hydrolyse entfernt.

11. Verfahren nach Anspruch 10, **dadurch gekennzeichnet**, daß man als eine geschützte Phosphitgruppe übertragende Verbindung Monochlor-(ß-cyanoethyl-N,N-diisopropylamino)-phosphit verwendet und mit $J_2/H_2O$/Lutidin oder mit $S_8/CS_2$/Pyridin oxidiert.

12. Verfahren nach Anspruch 10, **dadurch gekennzeichnet**, daß man als eine geschützte Phosphitgruppe einführende Verbindung Monochlor-(ß-Methyl-N,N-diisopropylamino)phosphin verwendet oder/und Nucleosidmethylphosphonamidite zur Oligonukleotidsynthese verwendet und mit $J_2/H_2O$/Lutidin oxidiert.

13. Verfahren nach einem der Ansprüche 10 bis 12, **dadurch gekennzeichnet**, daß man als zweiwertigen Alkohol ein geradkettiges oder verzweigtes, substituiertes oder unsubstituiertes Alkandiol oder Alkendiol mit einer Länge der unverzweigten Kette von 2 bis 6 C-Atomen verwendet.

14. Verfahren nach einem der Ansprüche 10 bis 13, **dadurch gekennzeichnet**, daß man als zweiwertigen Alkohol ein Alkandiol mit der Formel $HO-(CH_2)_n-OH$, wobei n eine ganze Zahl von 2 bis 6 ist, verwendet.

15. Verfahren nach Anspruch 14, **dadurch gekennzeichnet**, daß man als zweiwertigen Alkohol Ethylenglykol verwendet.

16. Verfahren nach einem der Ansprüche 10 bis 15, **dadurch gekennzeichnet**, daß man eine Verbindung der allgemeinen Formel II, bei der die Reste $R_1$, $R_2$ und $R_3$ jeweils einen Methylrest bedeuten, und als zweiwertigen Alkohol Ethylenglykol verwendet.

17. Verfahren nach einem der Ansprüche 10 bis 16, **dadurch gekennzeichnet**, daß man bei der Oligonukleotidsynthese an die Verbindung der allgemeinen Formel IV zuerst ein Thymidinmonophosphat und dann ein Adenosinmonophosphat ankondensiert.

18. Verfahren nach einem der Ansprüche 10 bis 17, **dadruch gekennzeichnet**, daß man zur Entfernung der Schutzgruppen mit wäßrigem Ammoniak bei erhöhter Temperatur hydrolysiert.

19. Verwendung von modifizierten Oligonukleotiden gemäß einem der Ansprüche 1 bis 9 zur Blockierung der Replikation oder/und Expression von Genen, die eine mit dem modifizierten Oligonukleotid komplementäre Sequenz aufweisen.

20. Verwendung nach Anspruch 19, **dadurch gekennzeichnet**, daß die Zielzellen durch Oncogene transformierte Zellen oder/und Virus-infizierte Zellen sind und man modifizierte Oligonukleotide verwendet, die zu einem Teil eines für die Virusvermehrung essentiellen Gens komplementär sind.

21. Verwendung nach Anspruch 17 und 18, **dadruch gekennzeichnet**, daß man zur Blockierung der Genreplikation oder/und Genexpression in Säugetierzellen ein modifiziertes Oligonukleotid, das an der 5′-Phosphatgruppe oder/und an einer oder mehreren weiteren Phosphatgruppen des Oligonukleotids durch einen Methylrest oder/und eine $S^-$-Gruppe substituiert ist, verwendet.

22. Verwendung nach Anspruch 21, **dadurch gekennzeichnet**, daß man bei Menschen und Tieren die Bestrahlung der Zellen extrakorporal vornimmt.

23. Verwendung nach Anspruch 21, **dadurch gekennzeichnet**, daß man bei der Behandlung einer Virus-infizierten Leukozyten-oder Lymphozyten-Fraktion bei Menschen und Tieren Blut entnimmt, das Blut extrakorporal bestrahlt und dann wieder dem Körper zuführt.

24. Verwendung nach den Ansprüchen 19 bis 23, **dadruch gekennzeichnet**, daß man zur Behandlung von HIV-infizierten Lymphozyten modifizierte Oligonukleotide verwendet, die zu einem Teil des Reverse-Transcriptase-Gens des HIV-Virus komplementär sind, und das Blut in mehreren Zyklen nach Teilentnahme extrakorporal bestrahlt.

25. Verwendung nach Anspruch 22 bis 24, **dadruch gekennzeichnet**, daß man das modifizierte Oligonukleotid dem bereits entnommenen Blut zugibt und dieses sodann extrakorporal bestrahlt.

# FIG.1

AM 5'-ENDE MODIFIZIERTES DESOXYOLIGONUKLEOTID
MIT KOVALENT GEBUNDENEM PSORALENDERIVAT

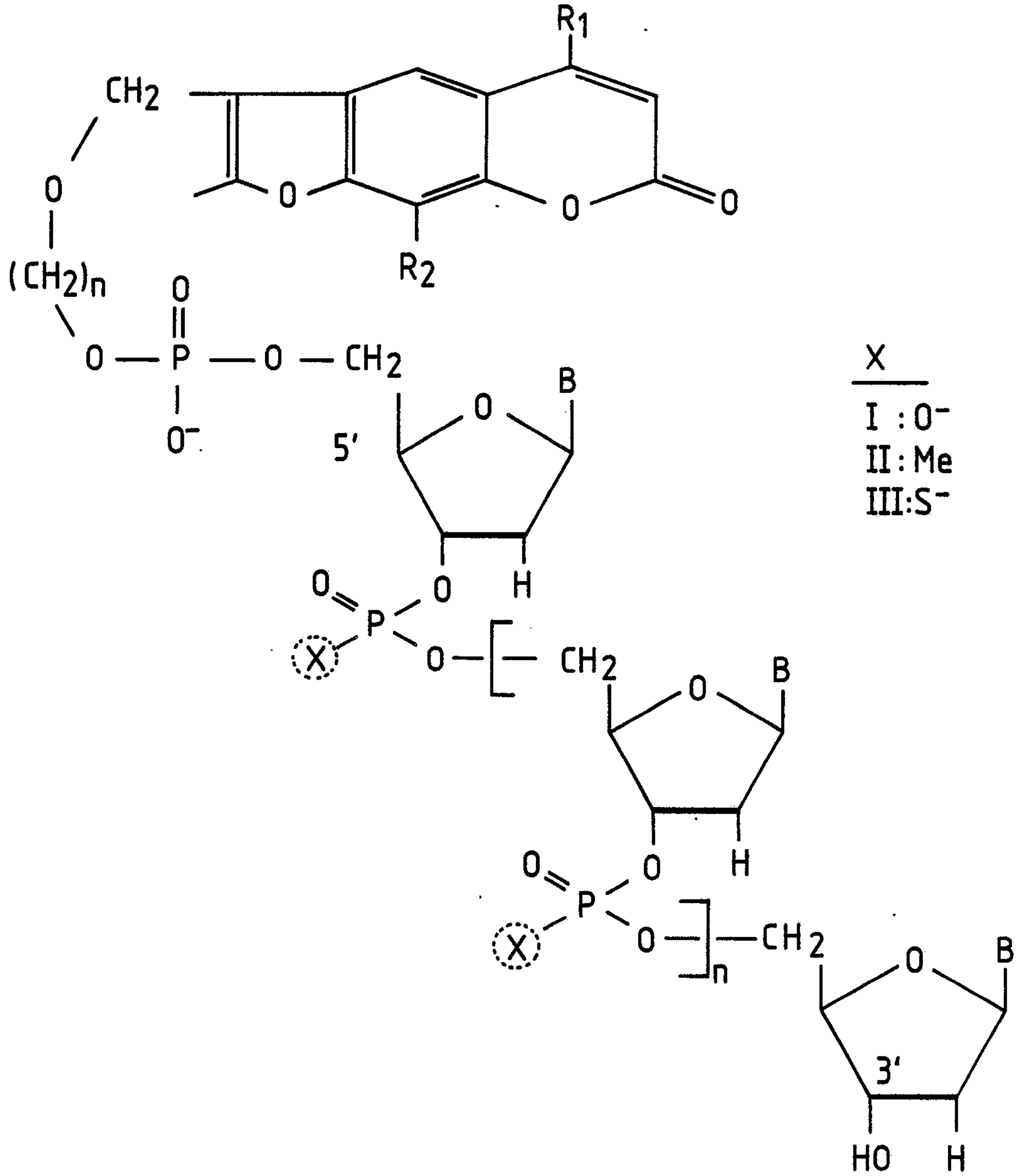

| X |
|---|
| I : $O^-$ |
| II : Me |
| III : $S^-$ |

FIG. 2

SYNTHESE VON 5'-MODIFIZIERTEN DEOXYOLIGONUKLEOTIDEN